# EUROPEAN PATENT APPLICATION

(11) **EP 1 291 438 A1**
(43) Date of publication of application: **12.03.2003**
(21) Application number: 01934548.7
(22) Date of filing: 05.06.2001
(51) Int. Cl.: C12Q 1/26, C12Q 1/37, G01N 33/68

(54) **METHOD OF DETECTING SACCHARIFIED ALBUMIN**

(30) Priority: 07.06.2000 JP 2000170773
(71) Applicant: BML, Inc., Tokyo 151-0051 (JP)
(72) Inventor: TSUBOI, Isami, c/o Laboratory BML, INC., Kawagoe-shi, Saitama 350-1101 (JP); TAGUCHI, Michihiro, c/o Laboratory, BML, INC., Kawagoe-shi, Saitama 350-1101 (JP)
(74) Representative: Bohmann, Armin K., Dr.
(86) International application number: JP0104734
(87) International publication number: WO01094618

(57) **Abstract**

The object of the present invention is to quantify glycated albumin, by providing means for providing quantification data, which correlate to the data of the quantification of glycated albumin by the HPLC method, without producing any intercept which might complicate use of the means in clinical medicine in a case where a regression curve is plotted by using the corrective quantification method with use of enzymes. Namely, the invention provides a method of detecting glycated albumin in the serum by using a reagent for assaying fructosamine, which comprises using a reagent for assaying glycated proteins by quantifying glycated proteins in the serum, correcting the value by dividing the thus-obtained value by the total albumin content in the serum and referring to the thus-corrected value as the glycated albumin content in the serum, wherein the method employs, as a glycated albumin standard, glycated albumin obtained from human blood by subjecting an albumin fraction originating in the blood of a healthy human to a treatment for distinguishing glycated albumin from non-glycated albumin.

## Description

### Technical Field:

The present invention relates to a method of assaying a specific glycated protein in serum.

### Background Art:

Treatment of diabetes mellitus is desirably carried out on the basis of diagnosis performed as early and as accurately as possible. As one marker employable for diagnosis of diabetes mellitus, serum glycated albumin has become of interest as an index for short-term blood sugar assessment. Thus, a suitable method for assaying glycated albumin is currently under investigation.

Presently, serum glycated albumin is determined by means of HPLC (*Rinshokensa,* vol. 40: 1275-1280, 1996). However, with HPLC, processing of a large number of specimens is difficult and operational cost is high.

The present inventors had previously performed extensive studies with an aim toward solving the above problems, and had conceived use of a glycoprotein assay reagent which is capable of assaying serum glycated protein through an enzymatic method. Moreover, in order to obtain improved accuracy from this assay scheme, they corrected a quantitative value of glycated protein obtained through use of the glycoprotein assay reagent. Specifically, they divided that value by a quantitative value of albumin which had been determined in the same serum sample, and found that such a corrected value exhibits very good correlation with a quantitative value of serum glycated albumin as determined through HPLC.

However, even when the above method which beneficially provides the mentioned good correlation is employed for quantitating glycated albumin, plotting of a regression curve produces a Y-intercept of negative value (or an X-intercept of positive value), thereby rendering handling cumbersome and inconvenient, and possibly impeding smooth processing in clinical or similar settings.

Accordingly, an object of the present invention is to provide means for eliminating the above-described intercept appearing in relation to plotting of a regression curve, as well as for establishing a 1:1 correlation between a value obtained through use of the enzymatic quantitation method with correction, and a corresponding quantitative value of glycated albumin obtained through HPLC.

### Disclosure of the Invention

In order to solve the above problems, the present inventors have first investigated the reason why the aforementioned intercept arises.

As a result, the inventors have found that the intercept arises from use of an artificial substance as a standard glycated albumin during the enzymatic quantitative assay.

The standard glycated albumin conventionally employed in the enzymatic assay is produced as follows. A human-derived albumin fraction or recombinant albumin obtained from *E. coli* which incorporates a gene that codes for albumin is incubated at 37°C or thereabouts in the presence of glucose, forcibly causing glycosylation of albumin, to thereby produce glycated albumin. Such artificially glycated albumin has been designed so as to cause glucose molecules to bind to all four sugar chain bonding sites present on the albumin molecule. However, when such glycated albumin produced through the conventional process is used as a standard substance in the enzymatic assay, the results do not always exhibit good correlation with those obtained through HPLC. A conceivable reason for this may be explained as follows. Inherently, the number of sugar molecules contained in one molecule of glycated albumin present in serum of a human so long as the serum is obtained from a healthy human is less than 4, and in many cases is 1 to 2 or thereabouts. Through any conventional HPLC-based quantitative technique for glycated albumin, irrespective of the number of sugar molecules bound to albumin (hereinafter the number is referred to as glucose binding number), recognition of one molecule of glycated albumin is possible. However, when quantitation is performed through the enzymatic method, the glucose binding number might affect the measurement. Also, if complete and thorough glycosylation is desired in the case where the above-mentioned forced glycosylation is performed (that is, when an average glucose binding number per molecule of glycated albumin is about 4), incubation in the presence of glucose must be performed for a prolonged period (for at least about one week), during which albumin tends to denature by itself. Thus, difficulty is encountered in incubation *per se* of albumin for such a considerable period of time. As a result, the distribution range of the glucose binding number per molecule of albumin is broadened, thereby permitting significant variation in measurements.

Thus, the results of enzymatic assay are inevitably different from the results obtained from the HPLC method, unless an employed standard substance of glycated albumin is a glycated albumin that simulates glycated albumin present in normal serum to the greatest possible extent and that exhibits reduced variation in the glucose binding number per molecule of albumin.

As means for eliminating disadvantages arising when artificial glycated albumin is used in the enzymatic assay, the present inventors have conceived separation/collection of glycated albumin present in (healthy) human serum and use of the same as a standard substance of glycated albumin.

Accordingly, the present invention provides a method of assaying glycated albumin through use of a glycoprotein assay reagent, in which glycated protein contained in a serum sample is quantitated with the glycoprotein assay reagent, and a quantitative value obtained therefrom is corrected by dividing the same by a quantitative value determined for total albumin in the same serum sample, and the thus-corrected value is considered a quantitative value of glycated albumin contained in that serum sample, wherein glycated albumin employed as a standard substance is glycated albumin obtained through separation from an albumin fraction derived from blood of a healthy human (hereinafter, the method may be referred to as the present assay method).

Glycated albumin derived from blood of a healthy human (in the present invention, the term "healthy human" refers to a person who is not afflicted with diabetes mellitus and who is not in the borderline stage of diabetes mellitus; more specifically, a "healthy human" is defined as a person whose fasting plasma glucose level is lower than 110 mg/dl, and whose plasma glucose level as measured 2 hours after a 75 g glucose tolerance test is lower than 140 mg/dl) generally signifies glycated albumin that is present in serum of a healthy human (hereinafter such glycated albumin may be referred to as natural glycated albumin), and specifically signifies such a glycated albumin containing sugar molecules in an average number of less than 4, typically 1 to 2, in one molecule of albumin (note: albumin has four binding sites available for binding of sugar molecules).

As described above, the natural glycated albumin which may be used as a standard substance in the present assay method generally contains 1 to 2, on average, sugar molecules per albumin molecule. As shown in Examples hereinbelow, a preferred glucose binding number per molecule of albumin is approximately 1.3 to 2.1 (rounded off to the nearest one-tenth). Therefore, the present invention also provides an assay method which employs as a standard substance a glycated albumin having an average glucose binding number of 1.3 to 2.1 (rounded off to the nearest one-tenth).

Accordingly, the present invention also provides a method of assaying glycated albumin through use of a glycoprotein assay reagent, in which glycated protein contained in a serum sample is quantitated with the glycoprotein assay reagent, and a quantitative value obtained therefrom is corrected by dividing the same by a quantitative value determined for total albumin in the same serum sample, and the thus-corrected value is considered a quantitative value of glycated albumin contained in that serum sample, wherein glycated albumin employed as a standard substance has an average glucose binding number of 1.3 to 2.1 per molecule of glycated albumin.

Effective, realistic manufacture of the mentioned natural glycated albumin may be attained through selective collection of the same by use of means for directly separating non-glycated albumin from glycated albumin contained in human serum. Such means may be a carrier to which aminophenylboronic acid has been immobilized as a ligand. However, the manufacturing method of the natural glycated albumin should not be understood as being limited only to this method; in one alternative method, a portion of sites available for sugar bonding in a non-glycated albumin is protected so as to prevent occurrence of glycosylation, and, under maintenance of such conditions, the non-glycated albumin is subjected to conventional forced glycosylation, followed by removal of the protective group, to obtain the natural glycated albumin.

Examples of the above-described glycoprotein assay reagent include a reagent prepared by combining protease and either ketoamino oxidase or fructosamine oxidase, which reagent exhibits substrate specificity for glycosylated amino acid. Products of this type are commercially available under the name "Autowako Fructosamine reagent" (product of Wako Pure Chemicals Industries, Ltd.) or "Gly-Pro™ Reagent" (product of Genzyme Diagnostics).

### Brief Description of the Drawings

Fig. 1 shows correlation between serum glycated albumin levels and corrected values obtained from the enzymatic method with correction, in which artificial glycated albumin was used as a standard substance; and
Fig. 2 shows correlation between serum glycated albumin levels and corrected values obtained from the enzymatic method with correction, in which natural glycated albumin was used as a standard substance.

### Best Mode for Carrying Out the Invention

Modes of the present invention will next be described in detail.

### A. Production of natural glycated albumin

As described above, at present, an effective method for producing the natural glycated albumin is separation and collection of the same substance from serum or plasma of a healthy human.

As a premise for producing the natural glycated albumin, preferably, healthy human albumin is prepared from a healthy subject. Human albumin, which contains glycated albumin, can be separated from human serum or plasma through a method known *per se.* For example, proteins contained in human serum or plasma are separated into albumin and other proteins by use of an anion exchange column, to thereby yield the target human albumin.

Subsequently, the thus-obtained human albumin of a healthy subject is preferably subjected to means capable of selecting non-glycated albumin or glycated albumin, to thereby separate and collect glycated albumin. One such means for selection may be use of a carrier employing, as a ligand, a molecular species which can adsorb specifically either glycated albumin or non-glycated albumin. For example, there may be employed a carrier bearing, as a ligand, aminophenylboronic acid immobilized thereto.

In a carrier in which aminophenylboronic acid has been immobilized as a ligand, the hydroxyl group of the immobilized aminophenylboronic acid is bonded to a cis-diol group of glucose of the glycated albumin, to thereby selectively capture glycated albumin. When the glycated albumin captured by the carrier is caused to elute, the natural glycated albumin of interest can be obtained. Exemplary means for eluting glycated albumin from a carrier bearing, as a ligand, aminophenylboronic acid in an immobilized form is substitution of glycated albumin that has been captured by the carrier by use of another cis-diol-containing substance, such as D-sorbitol. However, suitable elution means is not limited to the above, and should be selected depending on the specific carrier employed.

The above-described process for preparing glycated albumin may be used as means for selective production of non-glycated albumin, and the resultant non-glycated albumin also is very useful as a standard substance. Donors of blood from which non-glycated albumin is produced may be non-healthy humans, although they are preferably healthy humans. In general, when the above-described production process for glycated albumin is employed as means for the selective production of non-glycated albumin, the resultant non-glycated albumin typically contains natural glycated albumin in an amount of 10 to 15 mass% with respect to the mass of total albumin. When the present assay method is performed, serum is collected from a healthy human, and such serum itself may be used instead of the aforementioned non-glycated albumin.

Purification and collection of natural glycated albumin from a glycated albumin-containing fraction may be carried out by use of any suitable known method widely employed for purification and collection of proteins. Specifically, natural glycated albumin of interest can be purified and collected from a glycated albumin-containing fraction through, for example, treatment with a routinely employed protein precipitant, ultrafiltration, gel filtration, liquid chromatography, centrifugation, electrophoresis, or dialysis, in suitable combination as desired.

The thus-obtained natural glycated albumin has, on average, less than 4, typically 1 to 2, more specifically 1.3 to 2.1 (rounded off to the nearest one-tenth) glycosylation sites, per molecule of albumin, among four glycosylation sites in the albumin molecule, as described above.

As described above, through use of the natural glycated albumin as a standard substance for enzymatic-method-based quantitation of glycated albumin, there is no longer involved overreaction as observed in the existing enzymatic assay employing artificial glycated albumin, in which glycosylation has occurred at all the glycosylation sites. Therefore, while the simple and convenient feature of enzymatic-method-based quantitation, with correction, of glycated albumin is maintained, accurate and reliable data can be obtained.

Specifially, the present assay method ensures a nearly 1:1 correlation with quantitation results of glycated albumin through HPLC, and in addition, plotting of a regression curve does not produce an intercept.

### B. Present assay method and kit used therefor

The present assay method is premised on quantitation of glycated protein in serum (which may be prepared from a blood sample through a known method) through use of a glycoprotein assay reagent. An exemplary quantitation which may be employed is described by Young, D.C. in "Effects of Drugs on Clinical Laboratory Tests," 4th ed., AACC Press, Washington, DC, 1995.

The principle of the quantitation described therein may be summarized as follows.
1) Glycated protein in serum is digested by protease such as pronase or proteinase K, to thereby converting the same to fructosylamino acid.
2) Using the resultant fructosylamino acid as a substrate, and in the presence of oxygen, ketoamino oxidase or fructosamine oxidase is reacted, to thereby generate hydrogen peroxide.
3) The thus-produced hydrogen peroxide is quantitated (for example, through developing color with peroxidase in the presence of 4-aminoantipyrine and N-ethyl-N-(2-hydroxy-3-sulfopropyl)-m-toluidine, and measuring absorbance at 550 nm).

The present assay method is characterized in that the aforementioned natural glycated albumin is employed as a standard substance in such an enzymatic-method-based quantitation.

In the present assay method, total albumin must also be quantitated separately but in the same serum sample for which glycated protein is quantitated.

No particular limitation is imposed on the method for quantitating total albumin, and examples include immune nephelometry, latex method, and dye method.

Correction of data is performed by dividing the above-obtained quantitative value of glycated protein by the quantitative value of total albumin, to thereby precisely determine and quantitate the serum glycated albumin level.

According to the most preferred mode of the present invention, the following three steps are automated. 1) quantitation of serum glycated protein through use of the natural glycated albumin as a standard substance, 2) quantitation of serum total albumin, and 3) correction by dividing the value obtained from step 1) by the value obtained from step 2).

Such an automated process can be easily realized by persons having ordinary skill in the art by suitably adjusting or adapting an existing automated quantitation apparatus for serum samples to the present assay method.

The present invention provides, at low cost, test results which are equivalent to those obtained from conventional HPLC.

Depending on the specific purpose of the test, a urine sample may be used as a sample in the present assay method.

The present invention also provides an assay kit for performing the present assay method, comprising the natural glycated albumin (i.e., glycated albumin obtained through separation from an albumin fraction derived from blood of a healthy human, or glycated albumin having an average glucose binding number per mole of glycated albumin of 1.3 to 2.1) as a component of the kit (hereinafter, the kit may be referred to as the present assay kit). In addition to the aforementioned natural glycated albumin serving as a standard substance of the assay, according to needs, the present assay kit may include, as other components thereof, non-glycated albumin, and reagents for performing quantitation of glycated protein through the enzymatic method.

### Examples:

The present invention will next be described in detail by way of examples.

### Referential Example

Specimens were obtained from twenty blood donors (6 healthy subjects (specimen Nos. 1-6), 4 borderline diabetics (specimen Nos. 7-10), and 10 diabetics (specimen Nos. 11-20)), and for each specimen, percent by mass of glycated protein with respect to total albumin was determined by HPLC. From the thus-obtained measurements, mol ratio of glycated protein with respect to total albumin (this mol ratio is referred to as [A]) was calculated according to a known method ["Glycoalbumin," Keiko Yasukawa, *et al., Rinshokensa,* vol. 40 (12), pp 1275-1280 (1996)]. For the same specimen, mol ratio of serum glycated protein with respect to total albumin (this mol ratio is referred to as [B]) was also measured by use of a Gly-Pro™ reagent (product of Genzyme). The ratio [B]/[A] was calculated, to thereby compute an average glucose binding number per molecule of glycated albumin of each specimen. The results are shown in Table 1.

**Table 1**

| Sample No. | Ratio by mass (%) | [A] (µmol/g. ALB) | [B] (µmol/g. ALB) | [A]/[B] |
|---|---|---|---|---|
| 1 | 12.1 | 1.78 | 3.5 | 1.97 |
| 2 | 13 | 1.91 | 4 | 2.09 |
| 3 | 13.6 | 2 | 4 | 2.00 |
| 4 | 14.2 | 2.09 | 4.4 | 2.11 |
| 5 | 14.8 | 2.18 | 4 | 1.84 |
| 6 | 15.5 | 2.28 | 4.7 | 2.06 |
| 7 | 16.4 | 2.41 | 5.5 | 2.28 |
| 8 | 17.6 | 2.59 | 5.7 | 2.20 |
| 9 | 18.3 | 2.69 | 5.5 | 2.04 |
| 10 | 19.8 | 2.91 | 7 | 2.40 |
| 11 | 20.1 | 2.96 | 6.6 | 2.23 |
| 12 | 21.5 | 3.16 | 7.1 | 2.25 |
| 13 | 22.5 | 3.31 | 8.1 | 2.45 |
| 14 | 23.8 | 3.5 | 8.5 | 2.43 |
| 15 | 24 | 3.53 | 8.7 | 2.47 |
| 16 | 25.7 | 3.78 | 9.2 | 2.43 |
| 17 | 26.9 | 3.96 | 9.4 | 2.38 |
| 18 | 27.9 | 4.10 | 9.4 | 2.29 |
| 19 | 28.2 | 4.15 | 13.7 | 3.30 |
| 20 | 29.3 | 4.31 | 10.4 | 2.41 |

As is apparent from Table 1, the average glucose binding number per molecule of glycated albumin, as determined in each of specimen Nos. 1 to 6 collected from healthy donors, falls within the range of 1.3 to 2.1 (rounded off to the nearest one-tenth; average of the six specimen is 2.01), whereas the corresponding number as determined in specimens from the borderline diabetics or diabetics is higher and has a broader range.

In short, the glucose binding number per molecule of glycated albumin contained in blood of healthy humans distributes within a narrow range and is about 2, thereby proving that the healthy-human-derived glycated albumin is suitable as a standard substance in quantitation of glycated albumin through the enzymatic method.

### Preparation Example

Crude human albumin was separated from human serum (obtained from healthy donors of specimen Nos. 1-3 referred to in the above Referential Example; 150 mL × 3) by use of an anion exchange column and subjected to dialysis for removal of glucose, and the dialysate was concentrated. The resultant concentrate was diluted with saline, so as to adjust the protein concentration to 7 g/dl. Subsequently, the resultant human albumin solution was mixed with an aminophenylboronic acid-agarose gel (product of Sigma). Specifically, 5 mL of the human albumin solution was mixed with 5 mL of the gel (1:1 by volume). Prior to the mixing, the gel was treated with regeneration solution 1 (20mM sodium hydroxide) and regeneration solution 2 (0.3% acetic acid) and then equilibrated.

The thus-obtained gel mixture was left to stand in a refrigerator (4°C) for 16 hours, and thereafter, the serum present atop the gel was removed by aspiration with a pipette through the opening of the column.

### (1) Preparation of low-level glycated albumin specimen

An equilibrating solution (5 mL, 0.02M EPPS buffer (pH 8.6), 0.15M NaCl, 0.01M MgCl₂) was added to the gel mixture, to thereby induce elution of unadsorbed components. The solution containing the unadsorbed components was subjected to dialysis against cold saline having a volume 500 times that of the specimen, under stirring with a stirrer. The resultant dialysate was subjected to ultrafiltration by use of an ultrafiltration membrane (Centricon 10, product of Amicon) at 3,000 rpm for 6 hours, to thereby obtain a concentrate having about one-fourth the original volume, Centricon 10 being replaced with a new one when the volume was concentrated to one-half.

The thus-prepared concentrate served as a low-level glycated albumin specimen (non-glycated albumin).

Natural glycated albumin contained in the low-level glycated albumin specimen was found to be approximately 10 mass% with respect to the mass of total albumin.

### (2) Preparation of high-level glycated albumin specimen

The gel mixture from which unadsorbed components had been removed in the above step (1) was again washed with the above-mentioned equilibrating solution (45 mL). An eluant (20 mL, 0.02M EPPS buffer (pH 8.6), 0.15M NaCl, 0.1M sorbitol) was added to the gel mixture, and a fraction corresponding to 7 to 10 mL of the eluate was collected. Under stirring with a stirrer, the fraction was subjected to dialysis against cold saline having a volume 500 times that of the fraction. The resultant dialysate was subjected to ultrafiltration by use of an ultrafiltration membrane (Centricon 10, product of Amicon) at 3,000 rpm for 6 hours, to thereby obtain a concentrate having about one-fourth the original volume, Centricon 10 being replaced with a new one when the volume was concentrated to one-half.

The thus-prepared concentrate served as a high-level glycated albumin specimen (glycated albumin).

The average glucose binding number per molecule of albumin as determined in the high-level glycated albumin specimens was found to be 2.02.

### (3) Preparation of artificial high-level glycated albumin specimen

Crude human albumin (2 g) that had been separated from human sera (sera from donors of specimen Nos. 1-3) was dissolved in an aqueous solution (100 mL) containing 5 mass% glucose and 0.05 mass% sodium azide, and the solution was incubated at 37°C for 48 hours. Subsequently, the solution was subjected to dialysis against 5°C distilled water by use of a dialysis membrane overnight. After completion of dialysis, the dialysate was subjected to ultrafiltration by use of an ultrafiltration membrane (Centricon 10, product of Amicon) at 3,000 rpm for 6 hours, to thereby obtain a concentrate having about one-fourth the original volume, Centricon 10 being replaced with a new one when the volume was concentrated to one-half. The thus-obtained specimen served as an artificial high-level glycated albumin specimen.

### Test Example 1

Serum was obtained from each of the blood specimens collected from 48 donors, and serum glycated protein (1) was quantitated by use of a Gly-Pro reagent (Gly-Pro™ Reagent: product of Genzyme) and standard substances prepared in the above Preparation Example; i.e., the low-level glycated albumin specimen and the artificial high-level glycated albumin specimen.

Simultaneously, serum glycated albumin (2) was quantitated by means of HPLC (two-column method described in "*Rinshokensa*," vol. 40: 1275-1280, 1996).

Subsequently, value of (1) was divided by the total albumin level (g/L) which had been determined for each serum specimen by a conventional method, to thereby obtain a value (3).

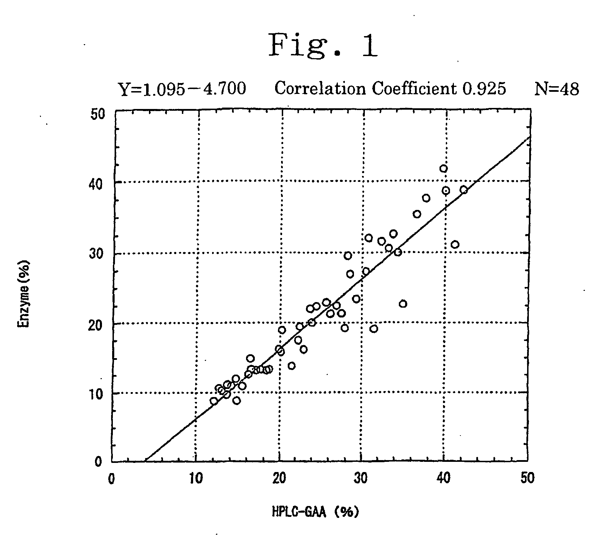

The value (3) was used as a substitute for value (1), and correlation between value (3) (%) and value (2) was studied. The results are shown in Fig. 1.

As shown in Fig. 1, a positive X-intercept is observed. The intercept is greater than the clinically accepted normal range as determined by HPLC. Therefore, use in clinical settings may result in confusion.

### Test Example 2

By use of a test system similar to that employed in Test Example 1, serum glycated protein (1)' was quantitated by use of standard solutions prepared in the above Preparation Example; i.e., the low-level glycated albumin specimen and the high-level glycated albumin specimen.

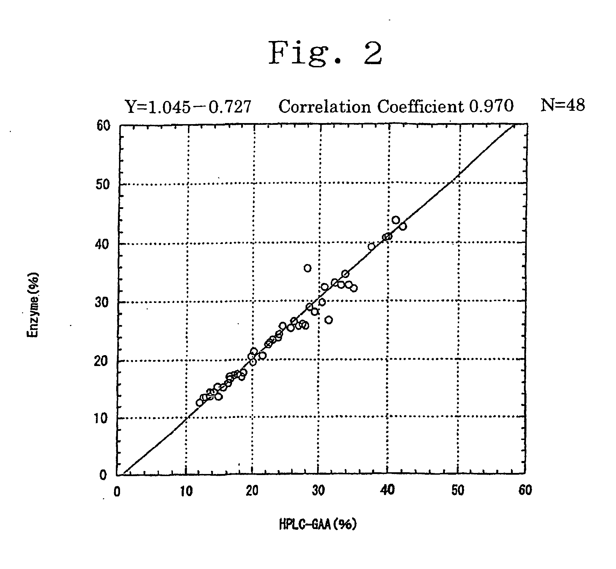

Correlation between the quantitative value of serum glycated albumin (2) in the same specimen through HPLC and a value (3)' obtained by dividing (1)' by the total albumin level was studied. The results are shown in Fig. 2.

As shown in Fig. 2, no such intercept as appearing in Fig. 1 is observed. The correlation coefficient and the slope were found to be 0.970 and 1.045, respectively, raising almost no variations from the measurements obtained through HPLC.

From these results, the present assay method has been confirmed to provide an accurate, convenient quantitation assay for glycated albumin, which can serve as a good index in relation to diabetes.

In conclusion, the present assay method is very effective means in diagnosis of diabetes.

### Industrial Applicability

The present invention has clarified that glycated albumin, which can serve as a good index in relation to diabetes, can be assayed or quantitated accurately and conveniently, and on the basis thereof, provides very useful means for detecting diabetes.

## Claims

1. A method of assaying glycated albumin through use of a glycoprotein assay reagent, in which glycated protein contained in a serum sample is quantitated with the glycoprotein assay reagent, and a quantitative value obtained therefrom is corrected by dividing the same by a quantitative value determined for total albumin in the same serum sample, and the thus-corrected value is considered a quantitative value of glycated albumin contained in that serum sample, wherein glycated albumin employed as a standard substance is glycated albumin obtained through separation from an albumin fraction derived from blood of a healthy human.

2. A method of assaying glycated albumin through use of a glycoprotein assay reagent, in which glycated protein contained in a serum sample is quantitated with the glycoprotein assay reagent, and a quantitative value obtained therefrom is corrected by dividing the same by a quantitative value determined for total albumin in the same serum sample, and the thus-corrected value is considered a quantitative value of glycated albumin contained in that serum sample, wherein glycated albumin employed as a standard substance has an average glucose binding number of 1.3 to 2.1 per molecule of glycated albumin.

3. Use of glycated albumin obtained through separation from an albumin fraction derived from blood of a healthy human as a standard substance in a method of assaying glycated albumin through use of a glycoprotein assay reagent, in which glycated protein contained in a serum sample is quantitated with the glycoprotein assay reagent, and a quantitative value obtained therefrom is corrected by dividing the same by a quantitative value determined for total albumin in the same serum sample, and the thus-corrected value is considered a quantitative value of glycated albumin contained in that serum sample.

4. Use of glycated albumin having an average glucose binding number of 1.3 to 2.1 per molecule of glycated albumin derived from a healthy human as a standard substance in a method of assaying glycated albumin through use of a glycoprotein assay reagent, in which glycated protein contained in a serum sample is quantitated with the glycoprotein assay reagent, and a quantitative value obtained therefrom is corrected by dividing the same by a quantitative value determined for total albumin in the same serum sample, and the thus-corrected value is considered a quantitative value of glycated albumin contained in that serum sample.

5. An assay kit for performing the method of assaying glycated albumin as described in claim 1, comprising, as a component of the kit, glycated albumin obtained through separation from an albumin fraction derived from blood of a healthy human.

6. An assay kit for performing the method of assaying glycated albumin as described in claim 2, comprising, as a component of the kit, glycated albumin having an average glucose binding number of 1.3 to 2.1 per molecule of glycated albumin.
